# EUROPEAN PATENT SPECIFICATION

(11) **EP 0 590 024 B1**
(45) Date of publication and mention of the grant of the patent: **02.05.1997**
(21) Application number: 92913363.5
(22) Date of filing: 19.06.1992
(51) Int. Cl.: A61K 45/06, A61K 31/245, A61K 31/255

(54) **TOPICAL COMPOSITION ENHANCING HEALING OF HERPES LESIONS**
Topisches Präparat zur besseren Heilung von Herpesverletzungen
COMPOSITION A USAGE LOCAL AMELIORANT LA CICATRISATION DE LESIONS DUES A L'HERPES

(30) Priority: 20.06.1991 US 718005
(43) Date of publication of application: 06.04.1994
(73) Proprietor: VIRO-TEX CORPORATION, The Woodlands, TX 77381 (US)
(72) Inventor: MILLER, Bruce, W., Tierra Verde, FL 33715 (US); KRONENTHAL, Richard, L., Fair Lawn, NJ 07410 (US)
(74) Representative: Dost, Wolfgang, Dr.rer.nat., Dipl.-Chem.
(86) International application number: US9205071
(87) International publication number: WO9300114

(56) References cited:
- EP-A- 0 305 551
- EP-A- 0 388 306
- EP-A- 0 394 928
- FR-A- 2 293 219
- FR-M- 4 579
- Journal of Pharmaceutical Sciences, vol. 75, no. 2, February 1986; P.P. SARPOTDAR et al.: "Evaluation of penetration enhancement of lidocaine by nonionic surfactants through hairless mouse skin in vitro", pages 176-181, see page 176, abstract

## Description

### Background of the Invention

Herpes simplex virus (HSV) causes a infection characterized by inflammation of skin and mucosal membranes of the mouth, and genitals. The type of HSV most commonly associated with lesions of the mouth ad facial region is Type I HSV. Type II HSV is most commonly associated with lesions of the genital region.

The symptoms of HSV generally include inflammation, pain and lesions. Symptoms initially include tingling, pain, and swelling at the site of infection, followed by lesions (cell death), healing in approximately ten to fourteen days. After the initial infection, recurrent attacks may occur throughout life in response to various stimuli, including changes in body temperature, stress, ultraviolet radiation, and exposure to chemicals.

Treatments for HSV are limited in efficacy. There is no known cure at this time, although various compositions are being marketed. Acyclovir (acycloguanosine) is administered systemically for treatment of HSV with some success. Topical acyclovir has been found to be somewhat effective for the primary lesion but not for recurrent lesions. Interferon has also been tested systemically and topically against HSV, but with only limited efficacy. There are so many side effects associated with systemic administration of antivirals that topical treatments are greatly preferred. Unfortunately, the topical administration even of the compounds having some efficacy when administered systemically is generally ineffective, so that the lesions are usually treated only with drying agents and/or topical aesthetics.

It is therefore a object of the present invention to provide topical compositions and methods of use thereof for the alleviation of the symptoms associated with herpes simplex infections or other viral lesions.

It is a further object of the present invention to provide topical compositions for alleviation of the symptoms of HSV infection with minimal side effects and relief from inflammation and pain normally associated with viral lesions.

EP-A-0 388 306 discloses a method for treating pain associated with herpes-zoster and post-herpetic neuralgia with a local anesthetic, e.g., lidocaine, and a vehicle for transdermal delivery of the medication, wherein nonionic surfactants may be employed. FR-M-45 79 relates to parenteral formulations for injection in which certain surfactants, e.g. quarternary ammonium salts, are administered with an active agent, e.g., lidocaine. FR-A-2 293 219 is directed to a method for inactivation of herpes virus by nonionic surfactants. Further, EP-A-0 305 551 discloses pharmaceutical compositions for treatment of herpes virus infections which compositions comprise antiviral nonionic surfactants in combination with interferon as the active ingredient.

### Summary of the Invention

Accordingly, the present invention provides a topical composition for treatment of lesions caused by herpes virus, as defined in claim 1.

Multiple daily applications of a composition having as the active ingredients an anesthetic and an anionic surfactant with antiviral activity decrease the time of healing of herpes simplex viral lesions from approximately ten days to five days, as well as decrease inflammation and pain. The composition was originally formulated as the combination of a commercially available topical cream with interferon containing tablets. The tablets contain 1,000 IU human leukocyte type A interferon in 1000 IU vitamin A, trace elements, and possibly catalase and superoxide dismutase activities. The interferon was added to the cream in a ratio of four tablets/one-half ounce by volume of cream. It has now been determined that the interferon is not an essential element of the composition. The anesthetic and the anionic surfactant are the effective in enhancing healing and minimizing the symptoms of the viral infection in the absence of the interferon. However, interferon may enhance their activity.

The composition is applied to the lesions daily, approximately every four hours, preferably beginning at the preliminary or prodromal stage of infection. Relief is almost immediate, and is characterized by decreased pain, swelling, and more rapid healing as compared with the untreated lesion.

### Detailed Description of the Invention

The formulation for alleviation of the symptoms of HSV lesions have two essential components, a topical or local anesthetic and an anionic surfactant, which are added to a conventional carrier cream, ointment, solution or gel containing appropriate stabilizers, buffers and preservatives for topical application to the lesion.

### Suitable Topical or Local Anesthetics.

The preferred anesthetic is tetracaine (either as the hydrochloride or other salt or as the free base). Topical anesthetics conveniently can be classified as esters, ethers or amides:

| Esters | Amides | Ethers |
|---|---|---|
| Tetracaine | Dibucaine | Dyclonine |
| Benzocaine | Lidocaine | Pramoxine |
| Proparacaine | | |
| Procaine | | |
| Cocaine (not a legal drug in the United States) | | |
| Butambin picrate (trinitrophenol 2-butyl-p-aminobenzoate) | | |

There are other anesthetics, used for injection, e.g., spinal anesthesia, which are not usually listed as topicals but are expected to work well on lesions where intact skin does not present a barrier. Some examples are:
- Chloroprocaine: Bupivacaine
Etidocaine
Mepivacaine
Prilocaine

Tetracaine is the preferred anesthetic because it is close in structure to procaine, the anesthetic first tested and demonstrated to penetrate skin effectively, it has *in vitro* viral anti-replication activity, and it is on the FDA monograph list which allows marketing as an over-the-counter (OTC) product for fever blisters and cold sores at concentrations between 1 and 2%, by weight.

In the preferred embodiment, the anesthetic is combined with carrier in a concentration range of between 1 and 2%, by weight, however, the anesthetic may be present in a concentration of between 0.15 and 15%, by weight.

### Surfactants.

Surfactants have been discovered to have potent antiviral activity *in vitro*, with a very low degree of toxicity. It is believed that the surfactant physically inactivates viruses and may facilitate penetration of the anesthetic to the cell receptors, increasing effectiveness of the compounds at concentrations not normally effective.

The material in the original formulation is sodium lauryl (or dodecyl) sulfate, referred to as "SLS", a common surfactant found in toothpaste and other cosmetic preparations, known to "unfold" the tertiary structure of proteins, but not previously known to have actual anti-viral activity. SLS is an anionic surfactant with a negatively charged sulfate group. SLS is also capable of penetrating intact skin very effectively.

Other sulfur containing surfactants, e.g., the sodium alkylaryl and aryl sulfonates and the alkyl sulfonates, can be used in place of SLS.

In the preferred embodiment, the surfactant is combined with carrier in a concentration range of between 0.5 to 2%, by weight; however, the surfactant may be present in a concentration of between 0.1 to 10%, by weight. Surfactants having different properties may also be combined to a concentration of between 0.1 to 20% by weight.

### Other Anti-Microbials.

The composition can contain other anti-microbials, including antibiotics, antifungals, and other antiviral compounds, which may complement or supplement the activity of the basic composition. Suitable antibiotics include tetracycline, polymyxin B or other common antibiotics used in topical compositions, especially over-the-counter formulations. Examples of useful antifungals are such as tolnaftate and micatin. Examples of antivirals include interferon, either natural or recombinant as well as nucleoside analogs. e.g., Acyclovir.

### Other components.

Counter-irritants such as camphor and menthol, drying agents such as benzyl alcohol, resorcinol, and phenol, and astringents such as zinc sulfate and tannic acid, can also be added to the composition.

Other types of agents can also be added to the formulation. For example, a sunscreen, e.g., PABA, can be added to the formula since it is known that cold sores can be triggered by ultraviolet radiation.

### Acceptable Pharmaceutical Carriers.

The composition can be prepared in almost any relatively inert topical carrier. Many such carriers are routinely used and can be obtained by reference to pharmaceutical texts. Examples include polyethylene glycols (PEG), polypropylene copolymers (pluronics), and some water soluble gels. The preferred carrier is an emulsified cream, but other common carriers such as certain petrolatum or mineral oil-based ointments in which the surfactant and anesthetic are dispersible can be substituted.

At this time, the preferred composition of the invention most effective in enhancing healing of viral lesions is: (all percents are by weight):
methyl paraben, 0.25 % (0.25 to 0.5%)
borax, 0.5 % (0.3 to 0.6%)
lauramide DEA, 4% (0.3 to 0.4%)
stearic acid, 20% (15 to 25%)
beeswax, 0.8% (0.5 to 1.5 %)
tetracaine, 1.8 % (1 to 2%)
sodium lauryl sulfate, 0.8 % (0.1 to 3%)
eucalyptus oil, 0.025 % (0.01 to 0.0.5%)
PCMX, 0.4% (0.3 to 0.5%)
water to a total weight of 100 grams

In most cases, it is preferred that the pH of the carrier containing the active ingredients is adjusted to a pH of 6 to 8, using as buffering agents ingredients such as the borax, although acceptable buffering agents could be used.

Additional additives may include antioxidants, fragrance, color, water, preservatives (either antioxidants or antimicrobials), lubricants, moisturizers, or drying agents.

### Method of Application.

The composition is applied by the patient to the lesions one to six times dally, most preferably beginning immediately after the prodrome is sensed. Enough composition is applied to cover the lesion or the prodromal area.

The formulation has been demonstrated to be active against both HSV 1 (cold sores and fever blisters) and HSV 2 (genital herpes). However, it may be active against other viruses including Herpes Zoster (causing Shingles) and the Varicella virus causing chicken-pox, which are similar in structure or mechanism of infection.

The present invention will be further understood by reference to the following examples.

### Example 1: Determination of the ability of selected components of the composition to directly inactivate or inhibit the replication of herpes simplex virus type-2.

### Cells.

A continuous line of human embryo lung cells (MRC-5) were grown in Eagle's minimal essential medium (MEM) in Earle's salts supplemented with 2 mM L-glutamine, 10 mg/ml gentamicin, 0.25 mg/ml amphotericin B (Fungisone) ad 10% heat-inactivated fetal calf serum (growth medium). Cells were grown at 37°C in a humidified 5% CO₂ atmosphere or in closed flasks. Maintenance medium was the same basic composition, except the concentration of fetal calf serum was 2%. Vero cells (continuous line of green monkey kidney cells) were grown in the same manner as MRC-5 cells.

### Viruses.

A clinical isolate from a genital lesion of Herpes simplex virus, type (HSV-2), was used.

Stocks of viruses were grown by infecting MRC-5 cells at multiplicity of 0.01 plaque forming units (PFU) per cell and harvested in maintenance medium. Virus was harvested by three cycles of freezing at -70°C and thawing, followed by centrifugation at 1000 x g at 4°C for 15 minutes to remove cell debris. Resulting virus stocks were aliquoted and frozen at -70°C.

### Chemicals.

Stock solutions of lidocaine hydrochloride, niacin, sodium lauryl sulfate, and tetracaine were prepared from commercially available reagents. Stock solutions were prepared in Eagle's MEM without supplements and stored at 4°C.

### Cytotoxicity studies.

Cytotoxicity assays were performed in MRC-5 cells and in Vero cells. The test compound was dissolved in maintenance medium at selected concentrations and inoculated onto MRC-5 or Vero cells in 96 well tissue culture plates. Each concentration was inoculated in triplicate. The plates were incubated at 37°C in 5% CO₂ and graded for cytotoxicity at 24,48 and 72 hours after inoculation. The highest concentration of compound showing 50% cytotoxicity after 72 hours was designated the end point.

The results are as follows:
**Procaine hydrochloride.** Procaine HCl exhibits significant cytotoxicity in MRC-5 cells at concentrations above 0.25% after 24 hr incubation. Procaine HCl was not as cytotoxic in Vero cells as it was in MRC-5 cells, showing significant toxicity only at concentrations above 2.0%.
**Tetracaine HCl.** Tetracaine HCl was also more toxic to MRC-5 cells, showing significant toxicity at concentrations above 0.03% at 24 hr, but not in Vero cells.
**Niacin.** Niacin is non-toxic in MRC-5 cells up to concentrations of 0.25%.
**Sodium lauryl sulfate (SLS**). SLS is highly toxic to MRC-5 cells at concentrations above 0.0025%.

### Inactivation Studies.

To determine if a test compound directly inactivates HSV-2, 0.5 ml of serial dilutions of the compound to be tested was mixed with 0.5 ml of stock HSV-2 (titer = 5.0 x 10 PFU/ml) and incubated at room temperature for 30 minutes. After 30 minutes, the compound-virus mixture was diluted 10,000 fold in maintenance medium and virus recovery was determined by inoculating MRC-5 cells. The cells were graded for cytopathic effect after 24 hr, 48 hr, and 72 hr.

**Procaine HCl.** Procaine HCl does not directly inactivate HSV-2 at the concentrations tested, up to 4%.

**Tetracaine HCl.** Tetracaine HCl does not directly inactivate HSV-2 at the concentrations tested, up to 2%.

**Niacin.** Niacin does not directly inactivate HSV-2 at the concentrations tested, up to 0.25%.

**Sodium Lauryl Sulfate.** SLS directly inactivates HSV-2 at concentrations ranging from 30% down to 0.02% and partially inactivates the virus at concentrations down to 0.0025%. No inactivation occurs at a concentration of 0.00032%.

### Antiviral Studies.

Antiviral activity, i.e., the ability of the compound to inhibit infection or replication of virus in cells, was measured by infecting confluent monolayers of MRC-5 cells in 12 x 100 mm culture tubes at a multiplicity of infection (MOI) of 3.0 plaque forming units (PFU) per cell of HSV-2 by removing the maintenance medium, washing the monolayers three times with phosphate buffered saline (PBS), and adding 0.1 ml of HSV-1 suspended in PBS. The multiplicity of infection was calculated by counting the number of viable cells in a duplicate tube and diluting the stock virus to produce a viral inoculum in a volume of 0.1 ml which contained three times the PFUs as the number of cells counted. After a 60 minute absorption period, the inoculum was removed and 4 mls of test compound at appropriate concentrations in maintenance medium or maintenance medium (control) was added. The tubes were incubated at 37°C for an additional 23 hours. At 24 hours post-infection, the medium was removed and the monolayers were washed twice with PBS and frozen and thawed 3 times in 1 ml of maintenance medium. Virus yields were determined by serial end-point titrations in 96-well tissue culture plates containing MRC-5 cells. All experiments were tested in triplicate. 50% end-points were calculated and titers were expressed in TCID 50 units. Antiviral effect was determined by subtracting the difference between the TCID 50s of the test compound and from the TCID 50s of HSV-2 virus stock titrated in the same experiment and calculated in percentages.

**Procaine HCl.** Concentrations of procaine HCl up to 2% were unable to inhibit the replication of HSV-2.

**Tetracaine HCl.** Tetracaine HCl partially inhibited the replication of HSV-2 at 0.25% and 0.5%. It is likely that higher concentrations of tetracaine HCl would completely inhibit the replication of HSV-2.

**Niacin.** Niacin does not inhibit the replication of HSV-2.

**Sodium lauryl sulfate.** SLS does not inhibit viral replication at concentrations of 0.0025% and 0.0013%.

In summary, the results show that SLS is the most active ingredient of those tested as an inactivator of HSV-2 at concentrations raging down to 0.02%, and that tetracaine HCl has some antiviral activity at a concentration of 0.5%, although procaine HCl does not. At high concentrations, SLS may also act by desquamation of infected cells in the lesions, causing their rapid removal by scavenger cells and washing, and enhanced healing as a result.

### Example 2: Evaluation of the in vivo efficacy of selected components of the composition on a primary genital herpes simplex virus type 2 infection of guinea pigs.

The antiviral activity of several compounds in a primary genital HSV-2 infection of guinea pigs was tested. The experiments were placebo-controlled and uninfected animals were treated with each of the preparations to assess skin irritation. Groups treated with 5% Acyclovir were included as positive controls.

### Material and Methods.

### Genital HSV-2 Infection of Guinea Pigs.

**Virus and Viral Inoculation.** The MS strain of HSV-2 was utilized to infect animals. Female Hartley strain guinea pigs, Charles River Breeding Laboratories, Kingston, NY, weighing 250 to 300 g were inoculated intravaginally with 1.4 x 10⁵ plaque forming units of HSV-2 one hour after being swabbed for the removal of vaginal secretions.

**Treatment of Guinea Pigs**. Groups of ten infected guinea pigs were treated topically, 0.1 ml intravaginally and 0.1 ml on external genital skin, three times daily, approximately every eight hours, for seven days, beginning either 6 hr or 48 hr after HSV inoculation. Groups of three uninfected animals were used to assess toxicity of each preparation.

**Simple Collection and Virus Assays.** To determine the effect of treatment on vaginal virus replication, swabs of vaginal secretions were obtained on days 1, 3, 5, 7 and 10 after HSV-2 inoculation, placed in a tube containing 2.0 ml of media, vortexed and frozen at -70°C until titrated for HSV-2. When all samples were collected, they were thawed, diluted serially and HSV-2 titers determined using a microtiter CPE assay in rabbit kidney cells. Mean peak virus titers and areas under the virus titer-day curves were calculated and analyzed.

**Scoring of External Genital Lesions.** To determine the effect of therapy on the development and spread of external genital lesions, lesion severity was scored on a 0-5+ scale through the primary infection (21 days). Mean peak lesion scores and the area under the lesion score-day curves were calculated and analyzed.

**Evaluation of Efficacy.** The number of animals infected over the number inoculated, lesion score-day areas and virus titer-day areas under the curve, peak lesion scores and peak virus titers between untreated and placebo-treated or placebo-treated and drug-treated animals were compared using the Mann-Whitney U rank sum test. A p-value of 0.05 or less was considered significant.

### Samples:

- 1A: base formulation without procaine
- 2A: base formulation without procaine or SLS
- 3A: base formulation without SLS, with 1.8% procaine
- 3: base formulation with 1.3% SLS and 1.8% procaine
- 5: base formulation without procaine, with 1.6% tetracaine and SLS
- 6: base formulation without procaine, with 1.6% tetracaine, interferon (Schering Plough, 200,000 units), and SLS

The base formulation is as defined for the preferred embodiment of the composition for enhancing heating of viral lesions, but without SLS or anesthetic. Acyclovir (ACV) was used as a positive control (i.e., having antiviral activity). Placebo (PBS) treated animals were compared with an untreated control animal. 2A was compared with the untreated animals and placebo-treated animals. ACV groups were compared to the placebo-treated group. All others were compared to 2A.

### Results:

For primary irritation determination, groups of three guinea pigs were treated with each of the samples or controls. No visible signs of any skin irritation or genital toxicity were observed. The animals remained healthy and normal in appearance throughout the study.

### Effect on viral replication.

After HSV-2 inoculation, viral replication in the vaginal tract reaches a peak on days 3 to 5, then declines gradually with most animals having cleared the virus by day 10. Only 5% ACV given 6 hr after viral inoculation significantly reduced the virus titer-day area under the curve (AUC) or the mean peak virus titer (p-values of 0.05 and <0.01, respectively). All other formulations failed to alter vaginal viral replication, regardless of when therapy was initiated.

### Effect on Lesion Development.

Three to four days after HSV-2 inoculation, vesicular lesions begin to appear on the external genital skin. Lesions progressed to an ulcerative stage by days 7 to 8 and gradually healed by days 15 to 21. Animals treated with the PBS placebo at +6 hr had a significantly increased lesion score-day AUC value when compared to the untreated control group (p-value of <0.05). The vehicle control for the formulations (2A) given at +6 hr had a significantly lower lesion score-day AUC value when compared to the untreated control group (P-value of <0.05). The vehicle control for the formulations (2A) given at +6 hr had a significantly lower lesion score-day AUC when compared to the +6 hr placebo and the untreated control groups (P-value of <0.05 and 0.001 respectively). However, 2A initiated 48 hr post-inoculation resulted in a significantly greater lesion score-day AUC when compared to both the +48 hr placebo and untreated control groups (P-values of <0.01 and 0.001 respectively).

Therapy with 1A, 5, 6 or 5% ACV initiated 6 hr following HSV-2 inoculation significantly decreased lesion development (P-values of <0.05 or less). ACV 5% also reduced the mean peak lesion score (P-value of <0.001). Therapy with these formulations at +48 hr failed to inhibit lesion development. Increased lesion score-day AUC's and mean peak scores were seen with 1A and 5 (P-values of 0.06 or less).

It is important to note that the placebo showed a lesion score of 42.5. Samples 1A, 3, 5, and 6, all containing 1.3% SLS, had scores of 17.2, 31.7, 19.8, and 17.4, respectively, while samples 2A and 3A, not containing SLS, had scores of 27.8 and 21.0, respectively. Samples 3 and 3A had atypical scores, believed to be due to errors in formulation or testing. However, it is evident that the presence of SLS in the formulation reduces the severity of the lesion course in this model.

While there is a large standard deviation in the results due to the animal model used in combination with the variation in solubility of components in the formulations, especially the non-SLS containing formulations, the average overall score of the SLS-containing samples is 12, as compared to 23 for the samples not containing SLS, a 48% reduction in severity. In the 24 hour group, the average scores are 14 for the SLS samples and 17.2 for the controls, an improvement of 19% , while in the 72 hour groups comparable scores of 10.5 versus 26 provide a 60% reduction in lesion severity.

### Example 3: Demonstration of in vivo efficacy in enhancing healing of HSV lesions in humans.

Case report data for six patients was obtained in a open trial using samples containing SLS and procaine. It was shown that the average number of days to healing was reduced to six from a historical mean of ten in the same patients. Furthermore, the course of the disease seemed to be altered favorably. The stages of infection are defined as:
- Erythema: (Ery)
- Papule: (Pap)
- Vesicle: (Ves)
- Edema: (Ede)
- Scab: (Sca)

Table 1 provides the results of a clinical study in which the cream was applied topically four times daily to a recurring (not primary) lesion:

**Table 1**

| **Clinical Study Results** | | | | | | | | |
|---|---|---|---|---|---|---|---|---|
| **Patient** | **Average Duration of Episode (Historical) Days** | **Infection Type** | **Number of Days** | | | | | **Episode Duration (days)** |
| | | | **Ery** | **Pap** | **Ves** | **Ede** | **Sca** | |
| 1 | 10 | genital | 4 | 0 | 0 | 0 | 0 | 5 |
| 2 | 12 | genital | 5 | 2 | 2 | 2 | 1 | 6 |
| 3 | 10 | labial | 5 | 2 | 0 | 0 | 0 | 5 |
| 4 | 10 | genital | 4 | 3 | 0 | 0 | 0 | 4 |
| 5 | 14 | genital | 6 | 0 | 0 | 0 | 0 | 7 |
| 6 | 6 | genital | 8 | 0 | 2 | 4 | 3 | 8 |
| | Average 10.3 days | | | | | | | Average 5.8 days |

It is important to note that four of the six patients never progressed beyond the papule state and that the average days to healing was reduced by 44%, as compared to historical controls which had been on systemic Acyclovir. Lesion-associated pain was reduced or eliminated in all cases.

### Example 4: Larger scale study in human patients.

33 subjects, 15 randomized to the placebo control (Placebo), and 18 subjects randomized to the active compound (Active), were treated for Herpes simplex I infection. The patients were characterized by the presence of herpes lesions on the lips and facial areas, which were predominantly of type I herpes simplex virus. Patients were instructed to apply the ointment/creme every two hours during waking hours, in an amount sufficient to cover the lesion, beginning within 48 hours of the prodromal stage until the scab falls off. The ointment contained, on a percent weight basis, 1.9% tetracaine and 1.0% sodium lauryl sulfate in an aqueous base of eucalyptus oil, stearic acid, lauramide DEA, PCMX, beeswax, methylparaben, and borax. Data on the physician's rating scales for pain, itching, healing and clinical effectiveness, as well as the patients' rating of the perceived benefit of the treatment, are shown in Table 2.

**Table 2**

| **Means, Standard Errors and Sample Sizes for Individual Rating Scales and Time in Days to Scab Formation and Scab Loss, by Treatment Group.** | | |
|---|---|---|
| **Rating Scales** | **Placebo Mean± S.E. (n)** | **Active Mean ± S.E. (n)** |
| **Pain** | 6.1 ± 0.95 (14) | 7.4 ± 0.64 (18) |
| **Itching** | 5.5 ± 0.99 (13) | 7.6 ± 0.63 (18) |
| **Healing** | 5.4 ± 1.01 (14) | 6.2 ± 0.79 (18) |
| **Effectiveness** | 5.9 ± 0.92 (14) | 6.9 ± 0.71 (18) |
| **Benefit** | 4.5 ± 0.83 (15) | 6.5 ± 0.78 (18) |
| **Scab Formation (Days)** | 2.4 ± 0.34 (14) | 2.8 ± 0.35 (18) |
| **Scab Loss (Days)** | 7.1 ± 0.66 (14) | 5.9 ± 0.29 (18) |

As can be seen from Table 2, the Active group had better ratings on all scales. By a one-tailed (Active better than Placebo) Wilcoxon Rank Sums Test, the perceived benefit was statistically significant (p=.043), while the rating for itching just failed to be significant (p=.055).

A Kaplan-Meier analysis of the distribution of times to scab loss was conducted. The two curves are significantly different by the Log-Rank test (p=.049), with the Active group having the shorter times.

The clinical implications of the preliminary statistical analysis are straightforward:
1. The major subjective variable (time until the scab falls off) is decreased from 7.1 to 5.9 days. This is approximately an 18% decrease in the healing time and is statistically significant (p=.049).
2. The objective evaluations by the investigators show itching to be on the verge of significance and positive trends for each of the other variables (pain, healing, overall effectiveness). It should be recognized that the investigator's opinion reflects to a certain degree the opinion of the patient and also his own negative bias towards topical treatment of herpes infections.
3. The patient benefit opinion is statistically significant (p=.043) and represents an important finding.
4. The question has been raised as to the non-responders. There are two types of non-responders; placebo-treated patients who have favorable opinions of the medication and active-treated patients who have unfavorable opinions. The variability in response in any clinical study for any disease is a natural phenomenon which is explained by the variability in human biology among different individuals. There are always non-responders in any study. In fact, a study would be highly suspect if there were not non-responders. This is even more apparent when subjective evaluations are used since there is no direct hard measurements.

However, in summary, the composition containing SLS and tetracaine appears to have given better results uniformly, with statistical significance on important measures in spite of the small sample.

## Claims

1. A topical composition for treatment of lesions caused by herpes virus comprising a pharmaceutically acceptable topical carrier and as the active agent, the combination of an anesthetic and an anionic surfactant to decrease the time of healing of said lesions.

2. The composition of claim 1 wherein the anesthetic is selected from tetracaine, dibucaine, dyclonine, benzocaine, lidocaine,pramoxine, proparacaine, procaine, trinitrophenol 2-butyl-p-aminobenzoate, chloroprocaine, bupivacaine, etidocaine, mepivacaine, and prilocaine.

3. The composition of claim 2 wherein the topical anesthetic is selected from tetracaine and procaine in a concentration of between 0.1 and 20 % by weight.

4. The composition of any of claims 1 to 3 wherein the anionic surfactant is selected from sodium lauryl sulfate, sodium alkyl aryl sulfonates, and alkyl sulfonates.

5. The composition of claim 1 wherein the anesthetic is tetracaine in a concentration of between about 0.2 and 2 % by weight and the surfactant is sodium lauryl sulfate in a concentration of between 0.5 and 5 % by weight.

6. The composition of any of the preceding claims further comprising compounds selected from antibiotics, antifungals, and antivirals other than the effective combination of anesthetic and anionic surfactant.

7. The composition of any of the preceding claims further comprising compounds selected from buffeting agents, antioxidants, preservatives, coloring agents, fragrances, lubricants, moisturizers, sunscreens, or drying agents.

8. The composition of any of the preceding claims wherein the carrier comprises stearic acid, lauramide DEA, sodium lauryl sulfate, borax, tetracaine, eucalyptus oil, beeswax, preservative, and methyl paraben.

9. Use of a composition comprising the combination of an anesthetic and an anionic surfactant in a pharmaceutically acceptable carrier for preparing a topical agent for treatment of lesions caused by herpes virus.

10. The use of claim 9 wherein the anesthetic or the anionic surfactant is as defined in any of claims 2 to 5.

11. The use of claim 9 or 10 wherein the composition further comprises one or more compounds as defined in claim 6 or 7.

12. The use of any of claims 9 to 11 wherein the composition is applied topically between one and six times daily to the lesions.

13. The use of any of claims 9 to 12 wherein the composition is applied to lesions infected with herpes simplex virus type I or type II.

14. A composition comprising the combination of an anesthetic and an anionic surfactant in a pharmaceutically acceptable carrier for use as a topical agent in a method for treatment of lesions caused by herpes virus.

15. The composition of claim 14, wherein the anesthetic or the anionic surfactant is as defined in any of claims 2 to 5.

16. The composition of claim 14 or 15 further comprising one or more compounds as defined in claim 6 or 7.

## Patentansprüche

1. Topische Zusammensetzung zur Behandlung von durch Herpesvirus verursachte Läsionen, enthaltend einen pharmazeutisch verträglichen topischen Träger und als Wirkstoff die Kombination eines Anästhetikums mit einem anionischen oberflächenaktiven Stoff, zur Verringerung der Abheilungszeit der Läsionen.

2. Zusammensetzung nach Anspruch 1, wobei das Anästhetikum aus Tetracain, Dibucain, Dyclonin, Benzocain, Lidocain, Pramoxin, Proparacain, Procain, Trinitrophenol-2-butyl-p-aminobenzoat, Chloroprocain, Bupivacain, Etidocain, Mepivacain und Prilocain ausgewählt ist.

3. Zusammensetzung nach Anspruch 2, wobei das topische Anästhetikum aus Tetracain und Procain in einer Konzentration zwischen 0,1 und 20 Gewichtsprozent ausgewählt ist.

4. Zusammensetzung nach einem der Ansprüche 1 bis 3, wobei der anionische oberflächenaktive Stoff aus Natriumlaurylsulfat, Natriumalkylarylsulfonaten und Alkylsulfonaten ausgewählt ist.

5. Zusammensetzung nach Anspruch 1, wobei es sich bei dem Anästhetikum um Tetracain in einer Konzentration zwischen etwa 0,2 und 2 Gewichtsprozent handelt und bei dem oberflächenaktiven Stoff um Natriumlaurylsulfat in einer Konzentration zwischen 0,5 und 5 Gewichtsprozent handelt.

6. Zusammensetzung nach einem der vorhergehenden Ansprüche, weiter enthaltend Verbindungen ausgewählt aus Antibiotika, Fungiziden und Antivirus-Mitteln, die von der wirksamen Kombination von Anästhetikum mit anionischem oberflächenaktivem Stoff verschieden sind.

7. Zusammensetzung nach einem der vorhergehenden Ansprüche, weiter enthaltend Verbindungen ausgewählt aus Puffersubstanzen, Antioxidationsmitteln, Konservierungsstoffen, Farbmitteln, Aromen, Gleitmitteln, Feuchthaltemitteln, Sonnenschutzmitteln oder Trockenmitteln.

8. Zusammensetzung nach einem der vorhergehenden Ansprüche, wobei der Träger Stearinsäure, Lauramid-DEA, Natriumlaurylsulfat, Borax, Tetracain, Eucalyptusöl, Bienenwachs, Konservierungsstoff und Methylparaben enthält.

9. Verwendung einer Zusammensetzung, enthaltend die Kombination eines Anästhetikums mit einem anionischen oberflächenaktiven Stoff in einem pharmazeutisch verträglichen Träger zur Herstellung eines topischen Mittels zur Behandlung von durch Herpesvirus verursachte Läsionen.

10. Verwendung nach Anspruch 9, wobei es sich bei dem Anästhetikum oder dem anionischen oberflächenaktiven Stoff um eines bzw. einen handelt wie in einem der Ansprüche 2 bis 5 definiert.

11. Verwendung nach Anspruch 9 oder 10, wobei die Zusammensetzung weiterhin eine oder mehrere Verbindungen wie in Anspruch 6 oder 7 definiert enthält.

12. Verwendung nach einem der Ansprüche 9 bis 11, wobei die Zusammensetzung zwischen ein- und sechsmal täglich topisch auf die Läsionen aufgetragen wird.

13. Verwendung nach einem der Ansprüche 9 bis 12, wobei die Zusammensetzung auf Läsionen aufgetragen wird, die mit dem Herpes-Simplex-Virus vom Typ I oder vom Typ II infiziert sind.

14. Zusammensetzung, enthaltend die Kombination eines Anästhetikums mit einem anionischen oberflächenaktiven Stoff in einem pharmazeutisch verträglichen Träger zur Verwendung als topisches Mittel in einem Verfahren zur Behandlung von durch Herpesvirus verursachte Läsionen.

15. Zusammensetzung nach Anspruch 14, wobei es sich bei dem Anästhetikum oder dem anionischen oberflächenaktiven Stoff um eines bzw. einen handelt wie in einem der Ansprüche 2 bis 5 definiert.

16. Zusammensetzung nach Anspruch 14 oder 15, weiterhin enthaltend eine oder mehrere Verbindungen wie in Anspruch 6 oder 7 definiert.

## Revendications

1. Une composition topique pour le traitement des lésions causées par le virus de l'herpès, comprenant un véhicule topique pharmaceutiquement acceptable et, comme principe actif, la combinaison d'un anesthésique et d'un tensio-actif anionique, pour diminuer le temps de cicatrisation desdites lésions.

2. La composition de la revendication 1, dans laquelle l'anesthésique est chois parmi la tétracaïne, la dibucaïne, la dyclonine, la benzocaïne, la lidocaïne, la pramoxine, la proparacaïne, la procaïne, le 2-butyl-p-aminobenzoate de trinitrophénol, la chloroprocaïne, la bupivacaïne, l'étidocaïne, la mépivacaïne et la prilocaïne.

3. La composition de la revendication 2, où l'anesthésique topique est choisi parmi la tétracaïne et la procaïne en une concentration comprise entre 0,1 et 20 % en poids.

4. La composition de l'une des revendications 1 à 3, où le tensioactif anionique est choisi parmi le laurylsulfate de sodium, les alkylarylsulfonates de sodium et les alkylsulfonates.

5. La composition de la revendication 1, où l'anesthésique est la tétracaïne en une concentration comprise entre environ 0,2 et 2 % en poids et le tensio-actif est le laurylsulfate de sodium en une concentration comprise entre 0,5 et 5 % en poids.

6. La composition de lune des revendications précédentes, comprenant en outre des composés choisis parmi les antibiotiques, les antifongiques et les antiviraux autres que la combinaison efficiente de l'anesthésique et du tensio-actif anionique.

7. La composition de l'une des revendications précédentes, comprenant en outre des composés choisis parmi les agents tampon, les antioxydants, les conservateurs, les agents colorants, les parfums, les lubrifiants, les humidifiants, les anti-écran ou les agents de séchage.

8. La composition de l'une des revendications précédentes, où le véhicule comprend l'acide stéarique, le DEA lauramide, le laurylsulfate de sodium, le borax, la tétracaïne, l'huile d'eucalyptus, la cire d'abeille, un conservateur et un parahydroxybenzoate de méthyle.

9. Utilisation d'une composition comprenant la combinaison d'un anesthésique et d'un tensio-actif de surface dans un véhicule pharmaceutique acceptable pour préparer un agent topique pour le traitement des lésions provoquées par le virus de l'herpès.

10. L'utilisation de la revendication 9, où l'anesthésique ou le tensio-actif de surface est tel que défini que dans l'une des revendications 2 à 5.

11. L'utilisation de la revendication 9 ou 10, où la composition comprend en outre un ou plusieurs composés tels que définis dans les revendications 6 ou 7.

12. L'utilisation de l'une des revendications 9 à 11, où la composition est appliquée topiquement entre une et six fois par jour sur les lésions.

13. L'utilisation de l'une des revendications 9 à 12, où la composition est appliquée à des lésions infectées par le virus de l'herpès simplex type I ou type II.

14. Une composition comprenant la combinaison d'un anesthésique et d'un tensio-actif anionique dans un véhicule pharmaceutiquement acceptable, pour une utilisation comme agent topique dans un procédé de traitement de lésions provoquées par le virus de l'herpès.

15. La composition de la revendication 14, où l'anesthésique ou le tensio-actif de surface est tel que défini dans l'une des revendications 2 à 5.

16. La composition de la revendication 14 ou 15, comprenant en outre un ou plusieurs composés tels que définis dans la revendication 6 ou 7.
